Europäisches Patentamt

European Patent Office

Office européen des brevets

(11) Publication number: **0 236 290**
**A1**

(12) **EUROPEAN PATENT APPLICATION**

(21) Application number: **87870030.1**

(22) Date of filing: **04.03.87**

(51) Int. Cl.⁴: **A 61 K 7/16**

(30) Priority: **05.03.86 US 836270**

(43) Date of publication of application:
**09.09.87 Bulletin 87/37**

(84) Designated Contracting States:
**BE DE ES FR GB IT**

(71) Applicant: **Monsanto Company**
**Patent Department 800 North Lindbergh Boulevard**
**St. Louis Missouri 63167 (US)**

(72) Inventor: **Kim, Keun Young**
**237 Ladue Lake Drive**
**St.Louis Missouri 63141 (US)**

(74) Representative: **Lunt, John Cooper et al**
**Monsanto Europe S.A. Patent Department Avenue de**
**Tervuren 270-272 Letter Box No 1**
**B-1150 Brussels (BE)**

(54) Anticalculus dentifrices.

㉗ Anticalculus dentifrices having soluble pyrophosphate or tripolyphosphate as an anticalculus agent, with calcium pyrophosphate utilized as an abrasive agent.

EP 0 236 290 A1

Bundesdruckerei Berlin

**Description**

ANTICALCULUS DENTIFRICES

BACKGROUND OF THE INVENTION

The invention of this application relates to anticalculus dentifrices.

A wide variety of chemical and biological agents have been suggested for incorporation into dentifrices to inhibit calculus formation on teeth and/or remove calculus after it has been formed. As early as 1937 British Patent 490,384 disclosed doral compositions containing ethylenediaminetetraacetic acid, nitrilotriacetic acid and related compounds as anticalculus agents. U.S. Patent 3,678,154 disclosed the use of polyphosphonates. U.S. patent 3,737,533 disclosed the use of certain carbonyl diphosphonates. U.S. Patent 3,542,917 disclosed the utility of a polyester of a polycarboxylic acid having three or more carboxylic groups and a polyalkalene ether having at least two hydroxylic groups as a calculus retarding agent. U.S. Patent 3,429,963 disclosed calculus removal by the use of copolymers of ethylene and maleic anhydride. U.S. Patent 3,488,419 disclosed the use of a phosphonic acid such as ethane-1-hydroxy-11-diphosphonic acid. U.S. Patents 4,105,756; 4,130,635; 4,143,125; and 4,151,271 disclosed a variety of ether polycarboxylates as anticalculus agents.

Because many of the suggested anticalculus agents are not commonly used in human ingestible preparations, the likelihood of their use as anticalculus agents in dentifrice preparations is not strongly favored absent extensive clinical testing for safety as well as efficacy. What would be desirable is the discovery that a compound generally recognized as safe for human ingestion would also have anticalculus efficacy. Such properties are found in a group of compounds designated as soluble pyrophosphates which have considerable history of safe use in human ingestible products and as recently disclosed in U.S. Patent 4,515,772 (incorporated herein by reference), soluble pyrophosphates exhibit some anticalculus efficacy in dentifrice preparations. Among the dentifrice formulations disclosed are those utilizing calcium pyrophosphate as an abrasive agent. However, in the file history of U.S. Patent 4,515,772, there is evidence that preparations of dentifrice formulations comprising calcium pyrophosphate as an abrasive agent and soluble pyrophosphates as an anticalculus agent are ineffective as anticalculus dentifrices. In this regard, the evidence of record suggests that a dentifrice with an efficaciously high level of soluble pyrophosphate cannot be achieved with calcium pyrophosphate incorporated into a dentifrice as an abrasive.

SUMMARY OF THE INVENTION

What has been discovered is that calcium pyrophosphate can surprisingly be utilized as an abrasive in a dentifrice employing soluble pyrophosphate as an anticalculus agent provided that the level of readily soluble calcium is low. It has also been discovered that polyphosphates such as tripolyphosphates are effective as anticalculus agents. By this invention, there is provided anticalculus dentifrices having soluble pyrophosphate or tripolyphosphate as an anticalculus agent even when calcium pyrophosphate is employed as an abrasive agent.

DETAILED DESCRIPTION OF THE INVENTION

Percentages reported herein are by weight, unless indicated otherwise; concentration of phosphates, such as pyrophosphates, tripolyphosphates, orthophosphates and the like are reported as equivalent $P_2O_5$. Temperatures are in degrees Celcius.

The dentifrices of this invention comprise as anticalculus agents soluble pyrophosphate or tripolyphosphates. Such pyrophosphates and tripolyphosphates are present as non-toxic salts of biologically compatible cations, such as alkali metal cations, preferably sodium or potassium. Tripolyphosphates are advantageous anticalculus agents in that hydrolysis products of tripolyphosphates include pyrophosphates and orthophosphates.

The dentifrices of this invention will contain a clinically effective amount of soluble pyrophosphate or tripolyphosphate as anticalculus agents to provide a demonstrable efficacy in retarding the deposit and/or removal of, calculus from the surfaces of teeth. Generally, the dentifrices of this invention will comprise soluble pyrophosphate or tripolyphosphate at a level of at least about 1% of the dentifrice, calculated on a weight basis of equivalent $P_2O_5$. In some instances, even lower amounts of anticalculus agent may prove efficacious. Generally the amount of anticalculus agent will not exceed about 10% of the dentifrioe.

The dentifrices of this invention will desirably be biocompatible and not adverse to mouth tissues. Such dentifrices will generally exhibit a pH in the range of about 5 to 9. Certain soluble pyrophosphates and tripolyphosphates, for instance tetra alkali metal pyrophosphates, will tend to provide a dentifrice having a high pH, for instance greater than about 8. A more desirable pH can be provided by adding to the dentifrice an acidulant, that is a compound comprising acid components that will tend to neutralize the effects of the more strongly basic pyrophosphates or tripolyphosphates. The acidulant can be added to provide a dentifrice having a more neutral pH or even a slightly acidic pH, for instance as low as about 5. Useful acidulants will include any biologically acceptable acid, for instance phosphoric acid or low pH salts, for instance mono- or di-alkali metal pyrophosphates, such as monosodium pyrophosphate or disodium pyrophosphate. The use of phosphoric acid is especially desirable also as a buffering agent to counteract acidity generated during hydrolysis e.g. acidity associated with the orthophosphate hydrolysis product of pyrophosphates and tripolyphosphates.

The dentifrices of this invention will generally comprise an abrasive, such as calcium pyrophosphate. In order to provide a dentifrice that will exhibit anticalculus effective levels of soluble pyrophosphate or tripolyphosphate after an extended period of time, for instance corresponding to the reasonable expected shelf life, it has been found that the abrasive should not contribute undesirable levels of calcium ion or other cations that form insoluble pyrophosphates. Cations, such as calcium ions, tend to interact with soluble pyrophosphates and/or polyphosphates resulting in a reduction in the amount of soluble pyrophosphate or tripolyphosphate to a level below a clinically effective amount. A calcium pyrophosphate useful as an abrasive in the dentifrices of this invention will be substantially devoid of soluble calcium salts that would provide the undesirably interactive calcium cations. An especially preferred form of calcium pyrophosphate comprises the predominantly beta phase e.g. at least about 50% beta phase calcium pyrophosphate, say about 60% or higher, e.g. 70%. Other abrasive agents that can be employed in dentifrices utilizing soluble pyrophosphate or tripolyphosphate as an anticalculus agent, include silicas, e.g. as disclosed in U.S. Patent 3,862,307, aluminas, and the like.

It is desirable in many cases to provide the dentifrices of this invention with a fluoride source, such as stannous fluoride, sodium fluoride, sodium monofluorophosphate, and the like. The amount of fluoride source incorporated into the dentifrice wil 1 generally provide fluoride ion in the range of about 50 to 3,500 ppm.

Other components of the dentifrices of this invention will be well known to those skilled in the art of formulating dentifrices and include, for instance, water; humectants such as glycerine, sorbitol, xylitol, propylene glycol; flavoring agents; sudsing agents such as alkyl sulfates, e.g. sodium lauryl sulfate; binders such as xantham gum, carrageenan, carboxymethylcellulose and the like; and antimicrobial preservatives, such as methylparahydroxybenzoate. The dentifrices of this invention are made using conventional mixing techniques, well known to those in the dentifrice industry.

The following examples further describe and demonstrate embodiments within the scope of the present invention. These examples are given solely for the purpose of illustration and are not to be construed as limitations of this invention. Many variations thereof are possible without departing from the true spirit and scope of this invention.

EXAMPLE 1

This example illustrates the results of screening tests of compounds as anticalculus agents. Such compounds included 1-hydroxyethylidene-1,1-diphosphonic acid (HEPD), ethylenediaminetetra methylenephosphonic acid (EDITEMPA), ethylenediamine tetraacetic acid (EDTA), sodium tripolyphosphate (STP), and tetrasodium tripolyphosphate (TSPP).

Each compound was evaluated as a potential anticalculus agent by a threshold test, a stabilization test and sequestration test.

The threshold test measured the minimum concentration (e.g., in ppm) of the compound required to inhibit calcium phosphate precipitation in vitro under simulated oral conditions at 37°C.

The stabilization test measured the effectiveness of the compound in inhibiting the hydrolysis of freshly precipitated calcium phosphate to stable calcium hydroxyapatite at pH 7.5 and 37°C; the results are reported as the volume (e.g. milliliters) of NaOH (e.g. 0.1N) required to maintain the pH at 7.5.

The sequestration test measures the sequestration power of the compound toward calcium ions. A standard solution of the compound is titrated into a standard calcium solution at pH 7. The results are reported as a change in potential in a calcium sensitive electrode (e.g. millivolts), indicating calcium ion depletion and as a minimum volume of compound solution required to effect the maximum amount of calcium sequestration as indicated by change in potential.

The results of screening are reported in Table 1 which indicates that sodium tripolyphosphate should exhibit anticalculus efficacy in dentifrices, as indicated by the relatively low threshold and stabilization test results and by desirably low sequestration power toward calcium.

## TABLE 1

| Materials | Threshold Test Concentration PPM | Stabilization Test Ml 0.1N NaOH | Sequestration Test mV | mL |
|---|---|---|---|---|
| HEDP | 8 | 6 | 36 | 12 |
| EDITEMPA | 5 | 8 | 44 | 14 |
| EDTA | 48 | 21 | 81 | 16 |
| STP | 10 | 10 | 60 | 10 |
| TSPP | 12 | 11 | 67 | 8 |

EXAMPLE 2

The following example illustrates embodiments of dentifrices according to this invention using calcium pyrophosphate as an abrasive.

## TABLE 2

| Component | Formulation, % | | | |
|---|---|---|---|---|
| | A | B | C | D |
| CPP | 42.0 | 39.2 | 42.0 | 42.0 |
| Glycerine | - | 25.0 | 25.0 | 25.0 |
| Sorbitol | 25.0 | - | - | - |
| CMC | 1.1 | 1.0 | 1.1 | 1.1 |
| TSPP | 2.5 | 5.0 | - | - |
| SAPP | 2.5 | - | - | - |
| STP | - | - | 5.0 | 5.0 |
| $H_3PO_4$ | - | 1.4 | 0.31 | 0.70 |
| Methyl Paraben | 0.1 | 0.1 | 0.1 | 0.1 |
| Propyl Paraben | 0.01 | 0.01 | 0.01 | 0.01 |
| SLS | 1.5 | 1.5 | 1.5 | 1.5 |
| Saccharin | 0.15 | 0.15 | 0.15 | 0.15 |
| Flavor | 1.0 | 1.0 | 1.0 | 1.0 |
| MFP | 0.75 | 0.76 | 0.76 | 0.76 |
| Water | balance | balance | balance | balance |
| pH | 7.1 | 7.1 | 8.5 | 7.1 |

Four toothpaste formulations were prepared from the following components in proportions indicated in Table II: calcium pyrophosphate (CPP) as an abrasive; glycerine (97% by weight in water) or sorbitol (70% by weight in water) as a humectant; carboxy methyl cellulose (CMC) as a binder; tetrasodium pyrophosphate (TSPP), disodium acid pyrophosphate (SAPP) or sodium tripolyphosphate (STP) as anticalculus agents; SAPP or phosphoric acid (85% by weight) as acidulants; methylparahydroxy-benzoate (methyl paraben) or propylparahydroxy-benzoate (propyl paraben) as antimicrobial preservatives; sodium lauryl sulfate (SLS) as a sudsing agent; saccharin as a sweetener; a flavoring agent; and sodium monofluorophosphate (MFP) as a fluoride source. The balance of each formulation was water. The pH of each formulation as prepared is also indicated in Table II.

The calcium pyrophosphate was beta phase rich and exhibited a Radioactive Dental Abrasion value of 610 as a powder and 476 when formulated in a toothpaste. Analysis of the calcium pyrophosphate indicated soluble phosphate in the range of 0.23 - 0.35% (distributed as 60% orthophosphate - 40% pyro- or polyphosphate). The calcium pyrophosphate in water (after 2 hours) exhibited a pH of 6.75.

Formulations were subjected to various accelerated aging tests to evaluate the stability of the anticalculus agent. Formulations A and B (as identified in Table II) were subjected to a five cycle freeze-thaw to determine any possible formation of the decahydrate of TSPP, a crystalline material often responsible for a gritty texture in toothpastes especially those having a pH greater than about 7.1. No grit was observed and the pH remained at 7.1. The formulations were subjected to high temperature aging as indicated in Table III to evaluate any reduction of anticalculus agent below a clinically effective level. For instance aging at 49°C for three weeks is believed to simulate on-the-shelf aging of about 2.7 years. The results indicated in Table III show residual amounts of soluble pyrophosphate and/or tripolyphosphate in the specified dentifrice both as a percentage by weight as $P_2O_5$ and as a percentage of the original. Available fluoride after each case of aging remained above 950 ppm as compared to initial fluoride level of about 1000 ppm.

## TABLE 3

### Residual Soluble Pyrophosphate and Tripolyphosphate, %
#### (% of Original)

| Aging | A | B | C | D |
|---|---|---|---|---|
| 5 days at 60° | 2.4 (79.2) | - | - | - |
| 3 weeks at 49° | 1.86 (61.4) | 2.02 (73.2) | 2.08 (64.5) | 1.93 (66.8) |
| 6 weeks at 49° | 1.17 (38.6) | - | - | - |

**Claims**

1. A dentifrice comprising essentially insoluble calcium pyrophosphate as an abrasive and a clinically effective amount of soluble pyrophosphate or tripolyphosphate as an anticalculus agent.

2. The dentifrice of claim 1 wherein said anticalculus agent, on a weight basis of $P_2O_5$, is at least about 1 percent of the dentifrice.

3. The dentifrice of claim 2 wherein said anticalculus agent, on a weight basis of $P_2O_5$, is about 1 - to about 10 percent of the dentifrice.

4. The dentifrice of claim 1 further comprising an acidulant.

5. The dentifrice of claim 4 wherein said acidulant comprises a mono- or di-alkali metal pyrophosphate or phosphoric acid.

6. The dentifrice of claim 1 wherein said anticalculus agent consists essentially of pyrophosphate and said dentifrice is water-based and has a pH no greater than about 7.1.

7. The dentifrice of claim 1 wherein said calcium pyrophosphate is substantially devoid of soluble calcium salts.

8. The dentifrice of claim 7 wherein said calcium pyrophosphate comprises a predominantly beta-phase calcium pyrophosphate.

9. The dentifrice of claim 1 further comprising a fluoride source.

10. The dentifrice of claim 9 wherein said fluoride source is sodium fluoride, sodium monofluorophosphate, or stannous fluoride.

11. A dentifrice comprising as an anticalculus agent sodium tripolyphosphate.

12. A dentifrice comprising an abrasive and an anticalculus agent provided by addition of sodium tripolyphosphate.

13. A method of making an anticalculus dentifrice comprising adding to said dentifrice an anticalculus effective amount of sodium tripolyphosphate.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 097 476 (THE PROCTER AND GAMBLE CO.) * page 5, lines 1-28; claim 1 * & US - A - 4 515 772 (Cat. D) | 1-10 | A 61 K 7/16 |
| A | US-A-3 137 632 (SCHIRALDI) * column 1, lines 63-72; column 2, examples 1-4 * | 1,11-13 | |
| A | BE-A- 637 092 (PROCTER AND GAMBLE CO.) * claim 3 * | 1-3 | |
| A | EP-A-0 155 440 (MONSANTO CO.) * claims 13, 18-20 * | 9-10 | |

TECHNICAL FIELDS SEARCHED (Int. Cl.4)

A 61 K 7/00
A 61 K 6/00

The present search report has been drawn up for all claims

| Place of search | Date of completion of the search | Examiner |
|---|---|---|
| BERLIN | 19-05-1987 | AVEDIKIAN P.F. |